# EUROPÄISCHE PATENTSCHRIFT

(11) **EP 1 201 642 B1**
(45) Veröffentlichungstag und Bekanntmachung des Hinweises auf die Patenterteilung: **10.08.2005**
(21) Anmeldenummer: 01124375.5
(22) Anmeldetag: 24.10.2001
(51) Int. Cl.: C07C 209/26, C07C 211/29

(54) **Verfahren zur Herstellung von Aminen durch reduktive Aminierung**
Process for the preparation of amines by reductive amination
Procédé de préparation d'amines par amination réductive

(30) Priorität: 27.10.2000 DE 10053380
(43) Veröffentlichungstag der Anmeldung: 02.05.2002
(73) Patentinhaber: BASF AKTIENGESELLSCHAFT, 67056 Ludwigshafen (DE)
(72) Erfinder: Liang, Shelue, Dr., 67071 Ludwigshafen (DE); Funke, Frank, Dr., 68161 Mannheim (DE); Höhn, Arthur, Dr., 67281 Kirchheim (DE)
(74) Vertreter: Isenbruck, Günter, Dr.

(56) Entgegenhaltungen:
- EP-A- 0 355 351
- EP-A- 1 035 106
- US-A- 2 298 284
- US-A- 4 929 737
- US-A- 5 554 573

## Beschreibung

Die Erfindung betrifft ein Verfahren zur Herstellung von Aminen durch katalytische hydrierende Aminierung von Carbonylverbindungen oder Alkoholen, die mindestens einen durch Halogen substituierten aromatischen Kern aufweisen, der bei der hydrierenden Aminierung nicht verändert wird.

Die hydrierende Aminierung von Carbonylverbindungen mit Ammoniak oder primären oder sekundären Aminen ist ein Standardverfahren zur Herstellung von Aminen. Üblicherweise wird die Umsetzung in Gegenwart von metallischen Katalysatoren durchgeführt, wobei beispielsweise Raney-Ni, Raney-Co, Pt/Aktivkohle, Pd/Aktivkohle, Pd/BaSO₄, Rh/Al₂O₃ als Katalysatoren zum Einsatz kommen. In Houben-Weyl, Methoden der Organischen Chemie, 4. Auflage, Band IV/1c, Reduktion Teil I, Thieme Verlag, 1980, Seiten 436 und 437 wird angegeben, dass aromatisch gebundenes Chlor bei der hydrierenden Aminierung mit Raney-Nickel nicht angegriffen wird. Es werden zwei beispielhafte Umsetzungen angegeben, die jedoch zu geringen Ausbeuten führen. Vermutlich trat bei den angeführten Beispielen eine Dehalogenierung als wesentliche Nebenreaktion auf.

In der GB 969,977 sind substituierte 1,3-Diphenyl-propylamine und entsprechende Imine beschrieben. Im Beispiel 6 wird die hydrierende Aminierung von 1-(2-Ethyl-4,5-dimethoxyphenyl)-3-(4-chlorphenyl)-1-propenol mit Ammoniak an Raney-Nickel als Katalysator beschrieben. Nach der Aufarbeitung wird 1-Amino-1-(2-ethyl-4,5-dimethoxyphenyl)-3-(chlorphenyl) propanhydrochlorid als Produkt erhalten.

Die EP-A-0 355 351 betrifft ein Verfahren zur Herstellung von Aminen durch reduktive Aminierung von Oxoverbindungen, die halogensubstituierte aromatische Substituenten enthalten. Die Umsetzung wird insbesondere an Raney-Ni oder Raney-Co in Gegenwart von Ammoniak oder Aminen durchgeführt. Zudem wird die Umsetzung in Gegenwart von organischen Schwefelverbindungen wie Dimethylsulfoxid oder Bis-(2-hydroxyethyl)-sulfid durchgeführt. Die organische Schwefelverbindung wird in einer Menge von vorzugsweise 1 bis 25 Gew. - %, bezogen auf den Katalysator, eingesetzt. In bezug auf die Carbonylverbindung liegen Mengen von mehr als 1 Gew.-% vor. Die Schwefelverbindungen dienen zur partiellen Vergiftung des Katalysators, so dass das aromatisch gebundene Halogen erhalten werden kann. Beispielsweise werden bei der Aminierung von p-Chloracetophenon unter geeigneten Bedingungen Ausbeuten an p-Chlorphenylethylamin von bis zu 90 % erzielt.

Der Zusatz der relativ großen Mengen an Schwefelverbindungen ist jedoch insofern nachteilig, da eine weitere Komponente in das Reaktionssystem eingeführt wird, die die Aufarbeitung der Reaktionsprodukte, insbesondere durch Destillation, erschweren kann. Zudem verursachen die organischen Schwefelverbindungen einen höheren Katalysatorverbrauch, der typischerweise über 6 Gew. - % des Ni-Katalysators, bezogen auf die eingesetzten Carbonylverbindungen, beträgt.

Aufgabe der vorliegenden Erfindung ist die Bereitstellung eines Verfahrens zur Herstellung von Aminen, die mindestens einen halogensubstituierten aromatischen Kern aufweisen, durch hydrierende Aminierung entsprechender Carbonylverbindungen oder Alkohole unter Erhalt der halogensubstituierten aromatischen Kerne, das die Nachteile der bekannten Verfahren vermeidet und in hohen Ausbeuten bei niedrigen Katalysatormengen zu den gewünschten Produkten führt.

Die Aufgabe wird erfindungsgemäß gelöst durch ein Verfahren zur Herstellung von Aminen der allgemeinen Formel (I)

R¹ R² CH-N R³ R⁴ (I)

in der R¹, R², R³ und R⁴ unabhängig voneinander Wasserstoff, geradkettiges oder verzweigtes C₁-C₁₂-Alkyl, C₃-C₁₂-Cycloalkyl, C₆-C₁₀-Aryl oder C₇-C₁₁-Aralkyl bedeuten, wobei mindestens einer der Reste R¹ und R² Aryl oder Aralkyl darstellt, das am aromatischen Kern mindestens einfach durch Halogen substituiert ist,
durch katalytische, hydrierende Aminierung von Carbonylverbindungen der allgemeinen Formel (II) oder Alkoholen der allgemeinen Formel (III)

R¹-C(=O)-R² (II)

R¹-CH(OH)-R² (III)

mit Stickstoffverbindungen der allgemeinen Formel (IV)

H N R³ R⁴ (IV)

mit den vorstehend genannten Bedeutungen für R¹ bis R⁴
in Gegenwart von Co- und/oder Ni-haltigen Katalysatoren, wobei die hydrierende Aminierung zusätzlich in Gegenwart von festen sauren Cokatalysatoren und in Abwesenheit von organischen Schwefelverbindungen durchgeführt wird.

Es wurde erfindungsgemäß gefunden, dass bei der hydrierenden Aminierung von Haloarylketonen, -aldeyhden oder -alkoholen auch ohne Zusatz organischer Schwefelverbindungen bei weitgehender Erhaltung des aromatisch gebundenen Halogens sehr gute Amin-Ausbeuten erzielt werden können, wenn man bei der Umsetzung die Co-und/oder Ni-haltigen Katalysatoren in Kombination mit festen sauren Cokatalysatoren einsetzt.

Die Co- und/oder Ni-haltigen Katalysatoren sind vorzugsweise ausgewählt aus Raney-Co, Raney-Ni, Raney-Co-Ni, Raney-Ni-Fe, Raney-Ni-Fe-Co, Ni-, Co-, Ni/Co-Mischoxiden oder Gemischen davon, die auch auf anorganischen Trägern vorliegen können.

Die Co- und/oder Ni-Katalysatoren können zudem zusätzlich 0 bis 80 Gew.-% Cu und 0 bis 10 Gew.-%, vorzugsweise 0 bis 5 Gew.-% weitere Metalle enthalten, berechnet als Metall und bezogen auf das Gesamtgewischt des Katalysators.

Im erfindungsgemäßen Verfahren werden die Co- und/oder Ni-haltigen Katalysatoren vorzugsweise in Form von elementarem Co- und/oder Ni-Schwamm, Raney-Cobalt oder Raney-Nickel, Co oder Ni bzw. Co-Oxid oder Ni-Oxid auf Trägern (Trägerkatalysatoren) verwendet. Sie können auch in beliebigen Gewichtsverhältnissen vermischt eingesetzt werden. Träger dafür sind z.B. Al₂O₃, SiO₂, TiO₂, ZrO₂, Aktivkohle und andere dem Fachmann bekannte Katalysatorträger.

Unter den Raney-Katalysatoren werden bevorzugt Raney-Katalysatoren wie Raney-Cobalt, Raney-Cobalt-Nickel, Raney-Cobalt-Nickel-Eisen, Raney-Cobalt-Nickel-Eisen-Chrom oder Raney-Cobalt oder Raney-Nickel mit Dotierungen anderer Übergangsmetalle in wasserfreier oder auch wasserfeuchter oder lösungsmittelfeucher Form eingesetzt. Besonders bevorzugt wird Raney-Cobalt, das jeweils 0 oder 0,1 bis 10; vorzugsweise bis 5 Gew.-% Al, Ni, Fe, Cr enthält, verwendet. Der Co- oder Ni-haltige Katalysator wird in einer Menge von 0,01 bis 20 Gew.-%, bevorzugt 0,1 bis 10 Gew.-% und besonders bevorzugt 0,3 bis 5 Gew.-%, bezogen auf die zu aminierende Carbonylverbindung, eingesetzt.

Es kann erfindungsgemäß auch vorzugsweise ein Co-Ni-Katalysator eingesetzt werden, der auf ZrO₂ geträgert ist und 0 bis 50 Gew.-% CuO, berechnet als CuO und bezogen auf das Gesamtgewicht der Katalysators, enthält.

Die festen sauren Cokatalysatoren sind erfindungsgemäß ausgewählt aus Oxiden oder Mischoxiden der Elemente Zr, Ti, Cr, Mo, W, Mn, Fe, B, Al, Si oder deren Gemische. Die Einsatzmengen der sauren Kontakte liegen vorzugsweise zwischen 0,1 und 20 Gew.-%, bevorzugt zwischen 1-10 Gew.-%, bezogen auf die zu aminierende Carbonylverbindung.

Beim Einsatz von Co- oder Ni-haltigen Trägerkatalysatoren können die Trägermaterialien wie Al₂O₃, SiO₂, TiO₂, ZrO₂ usw. als saure Kontakte fungieren. In diesem Fall ist die Zugabe eines zusätzlichen sauren Kontakts nicht nötig.

Im erfindungsgemäßen Verfahren werden als saure Kontakte vorzugsweise Metalloxide oder deren Mischungen eingesetzt. Besonders bevorzugt ist ZrO₂.

Die erfindungsgemäß eingesetzten Carbonylverbindungen oder Alkohole können weitgehend frei gewählt werden. Sie weisen mindestens einen Aryl- oder Aralkylrest auf, der am aromatischen Kern mindestens einfach durch Halogen substituiert ist. Vorzugsweise ist er durch Chlor substituiert, insbesondere durch ein Chloratom.

Besonders bevorzugt handelt es sich bei den Arylresten um Phenylreste und bei den Aralkylresten um Benzylreste. Die Alkylreste sind vorzugsweise C₁₋₆-Alkylreste, die Cycloalkylreste C₃₋₆-Cycloalkylreste. Besonders bevorzugt ist in den Verbindungen der allgemeinen Formel (II) und (III) keiner der Reste Wasserstoff. Insbesondere ist R¹ einfach durch Chlor substituiertes Phenyl, und R² ist C₁₋₆-Alkyl. Besonders bevorzugt wird p-Chloracetophenon als Carbonylverbindung eingesetzt. Bei der Stickstoffverbindung der Formel (IV) handelt es sich vorzugsweise um Ammoniak oder primäre oder sekundäre aliphatische Amine. Letztere weisen vorzugsweise C₁₋₆-Alkylreste auf. Besonders bevorzugt wird Ammoniak als Stickstoffverbindung eingesetzt.

Die Aminierung im erfindungsgemäßen Verfahren kann in Lösungsmitteln, beispielsweise Alkoholen wie Methanol, Ethanol, Propanol, Butanol, aliphatischen oder aromatischen Kohlenwasserstoffen wie Toluol, Xylol, Cyclohexan, Isooctan, Ether wie Tetrahydrofuran, Dioxan, Methyl-tert-butylether durchgeführt werden. In einer bevorzugten Durchführung wird jedoch kein zusätzliches Lösungsmittel verwendet, sondern das im Überschuss eingesetzte Aminierungsmittel (Ammoniak oder Amine) fungiert gleichzeitig als Lösungsmittel. Das Molverhältnis von Ammoniak oder Amin zu Carbonylverbindung liegt vorzugsweise bei größer 1, für schnelle Reaktionen vorzugsweise bei größer 2.

Die Aminierung wird vorzugsweise bei einer Temperatur von 10 bis 250°C, bevorzugt von 40 bis 150°C und bei einem Druck von 10 bis 200 bar, bevorzugt von 30 bis 130 bar durchgeführt.

Das erfindungsgemäße Verfahren kann im Festbett durchgeführt werden, wird aber z.B. in einem Autoklaven durchgeführt. Die Co- oder Ni-haltigen und die sauren Cokatalysatoren sowie die Carbonylverbindung werden unter Schutzgas in einer beliebigen Reihenfolge in den Reaktor eingefüllt. Bei Raumtemperatur werden Ammoniak oder Amine unter Rühren zudosiert. Die Mischung wird auf Reaktionstemperatur aufgeheizt, und anschließend wird Wasserstoff auf Reaktionsdruck aufgepresst. Nach Beendigung der Wasserstoff-Aufnahme wird der Reaktor auf Raumtemperatur abgekühlt, entspannt und entleert. Die Katalysatoren werden abfiltriert und für den nächsten Reaktionszyklus wieder eingesetzt. Die Aufarbeitung des Reaktionsproduktes erfolgt dann in dem Fachmann bekannter Weise.

Das Verfahren kann sowohl diskontinuierlich, beispielsweise in einem Autoklaven, wie auch kontinuierlich, beispielsweise in einem Druckrohr mit angeschlossenem Abscheider und Druckentspannung, durchgeführt werden.

Durch Verwendung von Co- und Ni-haltigen Katalysatoren in Kombination mit sauren Cokatalysatoren wird die Dechlorierung bei der hydrierenden Aminierung von Haloarylketonen und -aldehyden weitgehend unterdrückt, und dadurch können hohe Aminausbeuten erzielt werden. Die hohen Amin-Selektivitäten und Ausbeuten im erfindungsgemäßen Verfahren, kombiniert mit einfacher Durchführung der Synthese und Verzicht auf organische Hilfsmittel, ermöglichen ein einfache Aufarbeitung und damit insgesamt ein wirtschaftliches Herstellungsverfahren.

Das erfindungsgemäße Verfahren wird anhand folgender Beispiele mit p-Chloracetophenon als stellvertretende Carbonylverbindung näher erläutert.

### Beispiele:

Der in den Beispielen eingesetzte p-Chloracetophenon hat eine Reinheit von > 99% (lt. GC). Die dabei verwendeten Katalysatoren sind ebenfalls Handelsware mit folgender

| Zusammensetzung: | |
|---|---|
| Ra-Co | Raney-Cobalt, Suspensionskatalysator |
| Ra-Ni | Raney-Nickel, Suspensionskatalysator |
| Al₂O₃ | Pulver |
| ZrO₂ | Pulver |
| TiO₂ | Pulver |
| Al-Si | Al-Silikat, Pulver |
| Ni-Cu | NiO/CuO/MoO₃/ZrO₂ (Gew. 50,6 : 16,7 : 1,5 : 31,2) reduziert und teilpassiviert, zermahlen, Pulver |
| Ni-Cₒ | NiO/CoO/CuO/ZrO₂ (Gew. 28 : 28 : 13 : 31), reduziert und teilpassiviert, zermahlen, Pulver |

Die Aminierungsversuche wurden nach der unten stehenden allgemeinen Arbeitsvorschrift durchgeführt und sind nicht optimiert. Höhere Amin-Selektivitäten und -Ausbeuten sind daher möglicherweise erreichbar.

### Allgemeine Arbeitsvorschrift:

In einem mit Stickstoff gespülten und gefüllten 2,5 1 Autoklaven mit einem Scheibenrührer und zwei Wellenbrechern werden die Katalysatoren, p-Chloracetophenon und eventuell als Lösungsmittel Methanol vorgelegt. Bei Raumtemperatur werden unter Rühren (500 Umdrehungen/min) zunächst Ammoniak und dann Wasserstoff auf 20 bar aufgepresst. Die Mischung wird auf Reaktionstemperatur aufgeheizt, mit Wasserstoff auf Reaktionsdruck nachgepresst und anschließend unter konstantem Wasserstoff-Druck gerührt. Nach Beendigung der Wasserstoff-Aufnahme wird der Autoklav abgekühlt, entspannt, ausgegast und entleert. Die Zusammensetzungen der Reaktionsausträge werden gaschromatographisch bestimmt. Die Versuchsergebnisse sind in **Tab. 1** zusammengestellt.

**Tab. 1**

| Ergebnisse der Aminierung von p-Chloracetophenon (CAP) | | | | | | | | |
|---|---|---|---|---|---|---|---|---|
| Beisp. | Katalysatoren (g) | CAP/NH₃ MeOH [g] | T/P [°C/bar] | Ums. [%] | Amin [%] | Alk.^{a} [%] | SB^{b} [%] | Dechl.^{c} [%] |
| 1 | Ra-Co(10)/Al₂O₃(30) | 155 / 850 / 0 | 50 / 100 | 100 | 83,7 | 10,4 | 0 | 5,7 |
| 2 | Ra-Co(10) / Al-Si (30) | 155 / 850 / 0 | 50 / 100 | 99,3 | 68,0 | 27,0 | 0 | 2,7 |
| 3 | Ra-Co(5) / TiO₂(30) | 155 / 850 / 0 | 50 / 100 | 100 | 66,3 | 29,3 | 0 | 4,2 |
| 4 | Ra-Co(5)/ZrO₂(30) | 155/ 850/ 0 | 50 / 100 | 100 | 64,1 | 31,7 | 0 | 3,0 |
| 5 | Ra-Co(2,5) / ZrO₂(30) | 155 / 850 / 0 | 50 / 100 | 99,5 | 95,7 | 0 | 1,2 | 1,9 |
| 6 | Ra-Co(2,5) / ZrO₂(30) | 155 / 850 / 0 | 100 / 100 | 100 | 98,5 | 0,3 | 0 | 1,0 |
| 7 | Ra-Co(2,5)/ZrO₂(30) | 310 / 170 / 0 | 90 / 80 | 99,6 | 95,7 | 0,5 | 1,6 | 1,1 |
| 8 | Ra-Co(2,5) / ZrO₂(30) | 310 / 170 / 170 | 90 / 80 | 97,3 | 86,4 | 0,7 | 8,7 | 0,8 |
| 9 | Ra-Co(2,5)/TiO₂(30) | 310/ 170/ 0 | 90 / 80 | 74,1 | 25,0 | 0,2 | 45,7 | 0,2 |
| 10 | Ra-Ni(2,5)/ZrO₂(30) | 310 / 170 / 0 | 90 / 100 | 54,0 | 2,8 | 0,2 | 36,7 | 0,3 |
| 11 | Ra-Ni(2,5)/TiO₂(30) | 310 / 170 / 0 | 90 / 100 | 61,6 | 3,2 | 0,1 | 47,3 | 0,4 |
| 12 | Ra-Ni(2,5) / Al-Si(30) | 310 / 170 / 0 | 90 / 80 | 75,7 | 12,3 | 0,1 | 59,4 | 0,1 |
| 13 | Ni-Co(30) | 310 / 170 / 0 | 100/80 | 99,2 | 81,2 | 5,7 | 1,2 | 9,6 |
| 14 | Ni-Co(15) | 310 / 170 / 0 | 100 / 80 | 95,1 | 70,8 | 2,2 | 18,0 | 1,7 |
| 15 | Ni-Cu(30) | 310 / 170 / 0 | 100/ 80 | 97,5 | 82,8 | 1,5 | 7,7 | 3,1 |
| 16 | Ni-Cu(16) | 320 / 408 / 400 | 120/ 60 | 100 | 95,3 | 0,5 | 0,3 | 3,2 |

| | | | | | | | | |
|---|---|---|---|---|---|---|---|---|
| a. Alk. = 1-(4'-Chlorphenyl)ethanol; | | | | | | | | |
| b. SB = Schiff' sche Base, aus p-Chloracetophenon und p-Chlorphenylethylamin; | | | | | | | | |
| c. Dechl. = Summe der Dechlorierungsprodukte wie Phenylethylamin und 1-Phenylethanol. | | | | | | | | |

## Patentansprüche

1. Verfahren zur Herstellung von Aminen der allgemeinen Formel (I)
R¹R²CH-NR³R⁴ (I)
in der R¹, R², R³ und R⁴ unabhängig voneinander Wasserstoff, geradkettiges oder verzweigtes C₁-C₁₂-Alkyl, C₃-C₁₂-Cycloalkyl, C₆-C₁₀-Aryl oder C₇-C₁₁-Aralkyl bedeuten, wobei mindestens einer der Reste R¹ und R² Aryl oder Aralkyl darstellt, das am aromatischen Kern mindestens einfach durch Halogen substituiert ist,
durch katalytische, hydrierende Aminierung von Carbonylverbindungen der allgemeinen Formel (II) oder Alkoholen der allgemeinen Formel (III)
R¹-C(=O)-R² (II)
R¹-CH(OH)-R² (III)
mit Stickstoffverbindungen der allgemeinen Formel (IV)
H N R³ R⁴ (IV)
mit den vorstehend genannten Bedeutungen für R¹ bis R⁴
in Gegenwart von Co- und/oder Ni-haltigen Katalysatoren, **dadurch gekennzeichnet, dass** die hydrierende Aminierung zusätzlich in Gegenwart von festen sauren Cokatalysatoren und in Abwesenheit von organischen Schwefelverbindungen durchgeführt wird, wobei als feste saure Cokatalysatoror Oxide und Mischoxide der Elemente Qr,Ti,Cr,Mo,W,Mn,Fe,B,Al,Si oder dern Gemische verwendet werden.

2. Verfahren nach Anspruch 1, **dadurch gekennzeichnet, dass** die Co- und/oder Ni-haltigen Katalysatoren ausgewählt sind aus Raney-Co, Raney-Ni, Raney-Ni-Fe, Raney-Ni-Fe-Co, Ni-, Co-, Ni/Co-Mischoxiden oder Gemischen davon, die auch auf anorganischen Trägern vorliegen können

3. Verfahren nach Anspruch 1 oder 2, **dadurch gekennzeichnet, dass** die Co-und/oder Ni-haltigen Katalysatoren zusätzlich 0 bis 80 Gew. - % Cu und 0 bis 10 Gew. - % weitere Metalle enthalten, berechnet als Metall und bezogen auf das Gesamtgewicht des Katalysators.

4. Verfahren nach Anspruch 3, **dadurch gekennzeichnet, dass** ein Raney-CoKatalysator eingesetzt wird, der zusätzlich jeweils 0 bis 10 Gew. - % Al, Ni, Cr und/oder Fe enthält, berechnet als Metall und bezogen auf das Gesamtgewicht des Katalysators.

5. Verfahren nach Anspruch 3, **dadurch gekennzeichnet, dass** ein Co-Ni-Katalysator eingesetzt wird, der auf ZrO₂ geträgert ist und 0 bis 50 Gew. - % CuO enthält, berechnet als CuO und bezogen auf das Gesamtgewicht des Katalysators.

6. Verfahren nach einem der Ansprüche 1 bis 5, **dadurch gekennzeichnet, dass** die Katalysatoren und Cokatalysatoren in Suspension eingesetzt werden.

7. Verfahren nach einem der Ansprüche 1 bis 6, **dadurch gekennzeichnet, dass** in den Carbonylverbindungen der allgemeinen Formel (II) und den Alkoholen der allgemeinen Formel (III) R¹ einfach durch Chlor substituiertes Phenyl und R² C₁₋₆-Alkyl bedeuten.

8. Verfahren nach Anspruch 7, **dadurch gekennzeichnet, dass** p-Chloracetophenon als Carbonylverbindung und Ammoniak als Stickstoffverbindung verwendet werden.

## Claims

1. A process for preparing amines of the formula (I)
R¹R²CH-NR³R⁴ (I)
where R¹, R², R³ and R⁴ are each, independently of one another, hydrogen, straight-chain or branched C₁-C₁₂-alkyl, C₃-C₁₂-cycloalkyl, C₆-C₁₀-aryl or C₇-C₁₁-aralkyl, where at least one of the radicals R¹ and R² is aryl or aralkyl whose aromatic unit is substituted by at least one halogen,
by catalytic, hydrogenative amination of carbonyl compounds of the formula (II) or alcohols of the formula (III)
R¹-C(=O)-R² (II)
R¹-CH(OH)-R² (III)
by means of nitrogen compounds of the formula (IV)
HNR³R⁴ (IV)
where R¹ to R⁴ are as defined above,
in the presence of Co- and/or Ni-containing catalysts, in the additional presence of solid acid cocatalysts and in the absence of organic sulfur compounds, the solid acid cocatalysts used being oxides and mixed oxides of the elements Zr, Ti, Cr, Mo, W, Mn, Fe, B, Al, Si or mixtures thereof.

2. A process as claimed in claim 1, wherein the Co-and/or Ni-containing catalysts are selected from among Raney Co, Raney Ni, Raney Ni-Fe, Raney Ni-Fe-Co, nickel oxides, cobalt oxides, mixed Ni/Co oxides and mixtures thereof, which may also be present on inorganic supports.

3. A process as claimed in claim 1 or 2, wherein the Co- and/or Ni-containing catalysts further comprise from 0 to 80% by weight of Cu and from 0 to 10% by weight of other metals, calculated as metal and based on the total weight of the catalyst.

4. A process as claimed in claim 3, wherein a Raney Co catalyst which further comprises from 0 to 10% by weight of Al, Ni, Cr and/or Fe, calculated as metal and based on the total weight of the catalyst, is used.

5. A process as claimed in claim 3, wherein a Co-Ni catalyst which is supported on ZrO₂ and comprises from 0 to 50% by weight of CuO, calculated as CuO and based on the total weight of the catalyst, is used.

6. A process as claimed in any of claims 1 to 5, wherein the catalysts and cocatalysts are used in suspension.

7. A process as claimed in any of claims 1 to 6, wherein, in the carbonyl compounds of the formula (II) and the alcohols of the formula (III), R¹ is phenyl substituted by one chlorine atom and R² is C₁₋₆-alkyl.

8. A process as claimed in claim 7, wherein p-chloroacetophenone is used as carbonyl compound and ammonia is used as nitrogen compound.

## Revendications

1. Procédé de préparation d'amines de la formule générale (I) :
R¹R²CH-N R³R⁴ (I)
dans laquelle R¹, R², R³ et R⁴ représentent, indépendamment l'un de l'autre, de l'hydrogène ou un groupe alkyle en C₁-C₁₂ linéaire ou ramifié, cycloalkyle en C₃-C₁₂, aryle en C₆-C₁₀ ou aralkyle en C₇-C₁₁, au moins un des radicaux R¹ et R² représentant un groupe aryle ou aralkyle, qui est substitué au moins à une reprise par de l'halogène sur le noyau aromatique, par amination catalytique, hydrogénante, de composés carbonyle de la formule générale (II) ou d'alcools de la formule générale (III) :
R¹-C(=O)-R² (II)
R¹-CH(OH)-R² (III)
avec des composés d'azote de la formule générale (IV) :
H N R³R⁴ (IV)
où R¹ à R⁴ ont les significations précédemment citées,
en présence de catalyseurs contenant Co et/ou Ni, **caractérisé en ce que** l'amination hydrogénante est effectuée en supplément en présence de cocatalyseurs acides solides et en l'absence de composés de soufre organiques, des oxydes et oxydes mixtes des éléments Zr, Ti, Cr, Mo, W, Mn, Fe, B, Al, Si ou leurs mélanges étant utilisés comme cocatalyseurs acides solides.

2. Procédé suivant la revendication 1, **caractérisé en ce que** les catalyseurs contenant Co et/ou Ni sont choisis parmi du Co de Raney, du Ni de Raney, du Ni-Fe de Raney, du Ni-Fe-Co de Raney, des oxydes de Ni, de Co, des oxydes mixtes de Ni/Co ou leurs mélanges, qui peuvent aussi se présenter sur des supports inorganiques.

3. Procédé suivant la revendication 1 ou 2, **caractérisé en ce que** les catalyseurs contenant Co et/ou Ni contiennent en supplément 0 à 80% en poids de Cu et 0 à 10% en poids d'autres métaux, calculés sous la forme de métal et par rapport au poids total du catalyseur.

4. Procédé suivant la revendication 3, **caractérisé en ce qu'**on met en oeuvre un catalyseur à base de Co de Raney qui contient en supplément respectivement 0 à 10% en poids de Al, Ni, Cr et/ou Fe, calculés sous la forme de métal et par rapport au poids total du catalyseur.

5. Procédé suivant la revendication 3, **caractérisé en ce qu'**on met en oeuvre un catalyseur à base de Co-Ni qui est supporté sur ZrO₂ et contient 0 à 50% en poids de CuO, calculé sous la forme de CuO et par rapport au poids total du catalyseur.

6. Procédé suivant l'une des revendications 1 à 5, **caractérisé en ce que** les catalyseurs et cocatalyseurs sont mis en oeuvre en suspension.

7. Procédé suivant l'une des revendications 1 à 6, **caractérisé en ce que**, dans les composés carbonyle de la formule générale (II) et les alcools de la formule générale (III), R¹ représente du phényle substitué à une reprise par du chlore et R² un groupe alkyle en C₁-C₆.

8. Procédé suivant la revendication 7, **caractérisé en ce qu'**on utilise de la p-chloroacétophénone comme composé carbonyle et de l'ammoniac comme composé d'azote.
